# EUROPEAN PATENT APPLICATION

(11) **EP 4 674 368 A1**
(43) Date of publication of application: **07.01.2026**
(21) Application number: 24927628.8
(22) Date of filing: 13.05.2024
(51) Int. Cl.: A61B 18/12, A61B 18/14

(54) **PLASMA RADIOFREQUENCY SURGICAL ELECTRODE**

(30) Priority: 10.05.2024 CN 202410576437
(71) Applicant: Bangshimedicaltechnology Co., Ltd., Medical Device District Taizhou, Jiangsu 225300 (CN)
(72) Inventor: HE, Chengdong, Taizhou, Jiangsu 225300 (CN); YUE, Xin, Taizhou, Jiangsu 225300 (CN); JIN, Liangcui, Taizhou, Jiangsu 225300 (CN)
(74) Representative: Bayramoglu et al.
(86) International application number: PCT/CN2024/092759
(87) International publication number: WO 2025/231921

(57) **Abstract**

The present disclosure provides a radiofrequency (RF) plasma surgical electrode, and relates to the technical field of medical appliances. In order to solve the problem of an existing plasma surgical electrode that has a single surgical mode and cannot use a single electrode to achieve multiple modes of operation, thereby suffering from low surgical efficiency, the present disclosure proposes the following technical solution. The RF plasma surgical electrode includes a cable assembly, a proximal handle, a cutter shank and a distal cutter head that are connected in sequence, where the distal cutter head is internally provided with a first working electrode, a second working electrode and a bendable element; the bendable element and the cutter shank form a loop electrode; an RF plasma system, the first working electrode and the loop electrode form a first discharge loop to achieve a small-area strong cutting operation; and the RF plasma system, the second working electrode and the loop electrode form a second discharge loop to achieve a large-area cutting and hemostasis operation. The design solution of the present disclosure integrates multiple cutting functions and an innovative suck-while-coagulate function, significantly improving surgical efficiency, safety, and operational convenience, providing a more advanced and reliable tool for surgical operations.

## Description

### TECHNICAL FIELD

The present disclosure relates to the technical field of medical appliances, and in particular to a radiofrequency (RF) plasma surgical electrode.

### BACKGROUND TECHNOLOGY

Low-temperature plasma surgical systems have become a revolutionary technological breakthrough in the forefront exploration of medical technology. In recent years, low-temperature plasma surgical systems have shone brightly in the international medical field, and has especially demonstrated excellent therapeutic effects in clinical practice in many departments such as otolaryngology, spine surgery, gynecology, dermatology, and proctology. The widespread application of this innovative technology benefits from its significant advantages, including but not limited to high safety assurance, significant reduction in surgical time, minimization of trauma area, and rapid postoperative recovery. These characteristics collectively contribute to a dual improvement in patient treatment experience and efficacy. The operating mechanism of the low-temperature plasma system is based on the advanced electrosurgical technology, which achieves precise surgical intervention with the assistance of physiological saline by precisely regulating the plasma energy transmission process.

Specifically, the system utilizes the energy flow between the active electrode and the loop electrode to generate a dense and concentrated plasma vapor protective layer around the electrode periphery. The plasma vapor protective layer includes a large number of charged particles that move at high speeds. The kinetic energy of the charged particles is enhanced under the guidance of the electric field, thereby releasing sufficient energy to oxidize and decompose the molecular structure of the target tissue. Under a relatively low temperature of 40-70°C, the molecular bonds within tissue cells are effectively broken, promoting the rapid transformation of tissue materials into low-molecular-weight (LMW) molecules and atoms, thereby ensuring the immediacy and efficiency of tissue cutting and ablation processes and significantly reducing the risk of heat damage to surrounding healthy tissues.

However, despite the unprecedented surgical precision and safety brought by the low-temperature plasma technology, the prior art still has several limitations in the face of the challenges of complex anatomical structures. Especially for surgical areas that are obstructed by key physiological structures or bones and have special directions or are difficult to reach, traditional plasma surgical electrodes are often hard to achieve ideal surgical contact and operation, limiting the effective implementation of treatment. In addition, intelligent adjustment of energy output intensity based on the specific needs of different surgical sites to achieve optimal surgical results while ensuring surgical safety has become a key challenge that urgently needs to be overcome in the current research and development of electrosurgical instruments.

Given the above challenges, it is highly desirable to explore and develop a low-temperature plasma surgical system that can overcome anatomical barriers, precisely control energy output, and adapt to various complex surgical scenarios. This is an important direction for promoting medical technology innovation, and is an urgent need to meet higher clinical practice requirements and improve patient treatment outcomes.

### CONTENT OF THE INVENTION

An objective of the present disclosure is to provide a radiofrequency (RF) plasma surgical electrode. The present disclosure solves the problem of an existing plasma surgical electrode. That is, the existing plasma surgical electrode has a single surgical mode so that it cannot use a single electrode to achieve multiple modes of operation in accordance with different trauma intensity requirements, thereby suffering from low surgical efficiency.

In order to solve the above technical problem, the present disclosure adopts the following technical solution.

A radiofrequency (RF) plasma surgical electrode, including a cable assembly, a proximal handle, a cutter shank and a distal cutter head that are connected in sequence, where
the distal cutter head is internally provided with a first working electrode, a second working electrode and a bendable element; the bendable element and the cutter shank form a loop electrode; an RF plasma system, the first working electrode and the loop electrode form a first discharge loop; and the RF plasma system generates a plasma sheath at the first working electrode to achieve a small-area strong cutting operation; and
the RF plasma system, the second working electrode and the loop electrode form a second discharge loop; and the RF plasma system generates a plasma sheath at the second working electrode to achieve a large-area cutting and hemostasis operation.

In the present disclosure, the surgical electrode cutter head can achieve dual functions of small-area strong cutting and large-area cutting and hemostasis using the same device through the first working electrode, the second working electrode, and the loop electrode. Therefore, the present disclosure improves the efficiency and flexibility of surgery and reduces the need for changing instruments during surgery. The small-area strong cutting of the first working electrode is suitable for precise surgical operations, while the large-area cutting and hemostatic function of the second working electrode helps to quickly treat surgical wounds, reduce bleeding, accelerate the surgical process, and improve the success rate of surgery.

Furthermore, the proximal handle is internally provided with an adjustment assembly for adjusting a bending angle of the bendable element in a damping manner; the bendable element is internally provided with a cutting type spring tube communicated with the cutter shank; and the cutting type spring tube forms a suction hole at an outer edge of the distal cutter head.

Through the introduced adjustment assembly, especially the design of the damping adjustment, the surgeon can precisely adjust the angle of the bendable element according to surgical needs, thereby achieving precise positioning and operation of the cutting site, improving the precision and safety of surgery.

Furthermore, the adjustment assembly includes a lever provided on a handle housing, a damping partition element connected to the lever, and a wire reel matched with the damping partition element; and a traction wire is provided on the wire reel to pull the bendable element to bend the distal cutter head.

The combination design of the lever, the damping partition element and the wire reel simplifies the operation process of bending the cutter head, allowing the surgeon to easily control the cutter head shape, thereby reducing surgical fatigue and improving the surgical experience.

Furthermore, the cable assembly includes a cable connected to the RF plasma surgical system and a suction tube connected to a negative-pressure suction device; the cable is electrically connected to the loop electrode; the suction tube is separately communicated with the cutter shank and the cutting type spring tube; the first working electrode spans an outer edge of the suction hole; and the second working electrode surrounds the suction hole.

The cable assembly includes the cable connected to the RF plasma system, as well as the suction tube of a negative-pressure suction device, which realizes various functions such as cutting, coagulation, and suction, simplifies the surgical process, and reduces the space occupation of the operating room.

Furthermore, an outer edge of the distal cutter head further includes an electrode holder; and the electrode holder separates the first working electrode, the second working electrode, and the loop electrode.

The setting of the electrode holder effectively separates each electrode, avoiding unnecessary interference between electrodes and enhancing the safety of surgery.

Furthermore, the cutter shank includes an inner cutter shank and an outer cutter shank sleeved onto the inner cutter shank; an extension end of the inner cutter shank is connected to the cutting type spring tube; and the outer cutter shank and the bendable element form the loop electrode.

Furthermore, an end of the proximal handle adjacent to the cutter shank is provided with a cutter shank connector and a fastener for fixing the outer cutter shank.

Furthermore, the inner cutter shank is wrapped in an inner insulation layer, and the outer cutter shank is wrapped in an outer insulation layer.

The design of the inner and outer cutter shanks and the inner and outer insulation layers ensures the stability of current conduction, avoids current leakage during the surgical process, and protects the safety of patients and medical staff.

Furthermore, the damping partition element is provided with a plurality of damping grooves arranged at an interval; the wire reel is provided with damping bosses; and different damping bosses are matched with the damping grooves to achieve damping adjustment.

The present disclosure has the following beneficial effects.

In the present disclosure, the bendable cutter head design effectively solves the cutting and coagulation operations of traumas at different angles of the spine or joints, achieving trauma cutting and suck-while-coagulate operations, and maintaining a clear surgical field. Through the setting of the first working electrode and the second working electrode, the surgical electrode cutter head can achieve two different modes of small-area strong cutting and large-area cutting and hemostasis using the same device. The design improves surgical efficiency and flexibility, reduces the need for instrument replacement during surgery, and enables efficient cutting and coagulate-while-cut operations.

The combination of the inner cutter shank and the cutting type spring tube, as well as the design of the loop electrode formed by the outer cutter shank and the bendable element, improves the mechanical strength and durability of the surgical cutter head. In addition, the suction hole formed by the cutting type spring tube can effectively remove blood and tissue debris from the surgical area, maintaining a clear surgical field of view (FOV) and improving surgical comfort.

### DESCRIPTION OF THE DRAWINGS

FIG. 1 is a structural diagram of a radiofrequency (RF) plasma surgical electrode according to the present disclosure;
FIG. 2 is a structural diagram of an electrode handle according to the present disclosure;
FIG. 3 is an internal structural diagram of a wire reel of the handle according to the present disclosure;
FIG. 4 is a structural diagram of a cutter head according to the present disclosure;
FIG. 5 is a structural diagram of a damping partition element according to the present disclosure; and
FIG. 6 is a sectional view of the cutter head according to the present disclosure.

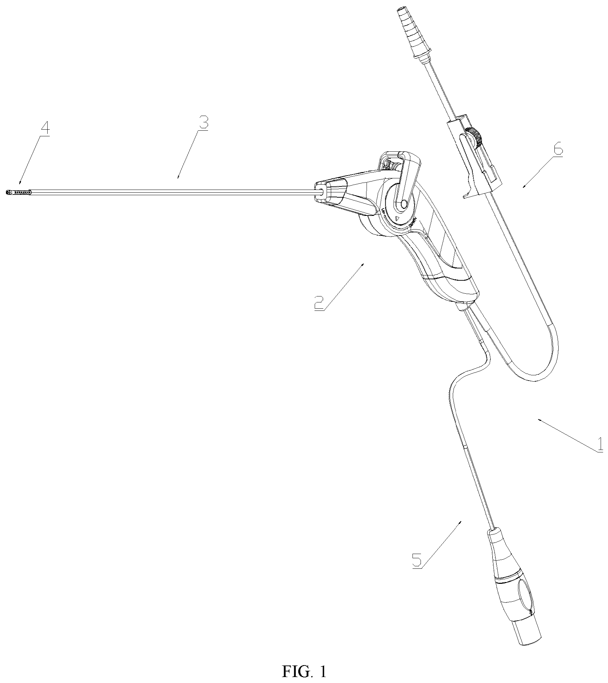

Reference numerals in FIGS. 1 to 6 are as follows: 1. cable assembly; 2. proximal handle; 3. cutter shank; 4. distal cutter head; 5. cable; 6. suction tube; 7. lever; 8. bendable element; 9. handle housing; 10. wire reel; 11. traction wire; 12. cutter shank connector; 13. fastener; 14. wire hole; 15. damping partition element; 16. first working electrode; 17. second working electrode; 18. electrode holder; 19. suction hole; 20. outer cutter shank; 21. inner cutter shank; 22. outer insulation layer; and 23. inner insulation layer.

### SPECIFIC IMPLEMENTATIONS

The technical solutions of the present disclosure are described clearly and completely below with reference to the drawings. Apparently, the described embodiments are merely a part rather than all of the embodiments of the present disclosure. All other embodiments obtained by those of skill in the art based on the embodiments of the present disclosure without creative efforts should fall within the protection scope of the present disclosure.

As shown in FIG. 1, the present disclosure provides a radiofrequency (RF) plasma surgical electrode, which is a medical surgical tool that integrates efficient cutting and precise hemostasis functions. The electrode cutter head system is designed with precision, including cable assembly 1, proximal handle 2, cutter shank 3, and distal cutter head 4, achieving comprehensive optimization from energy transmission to operational flexibility.

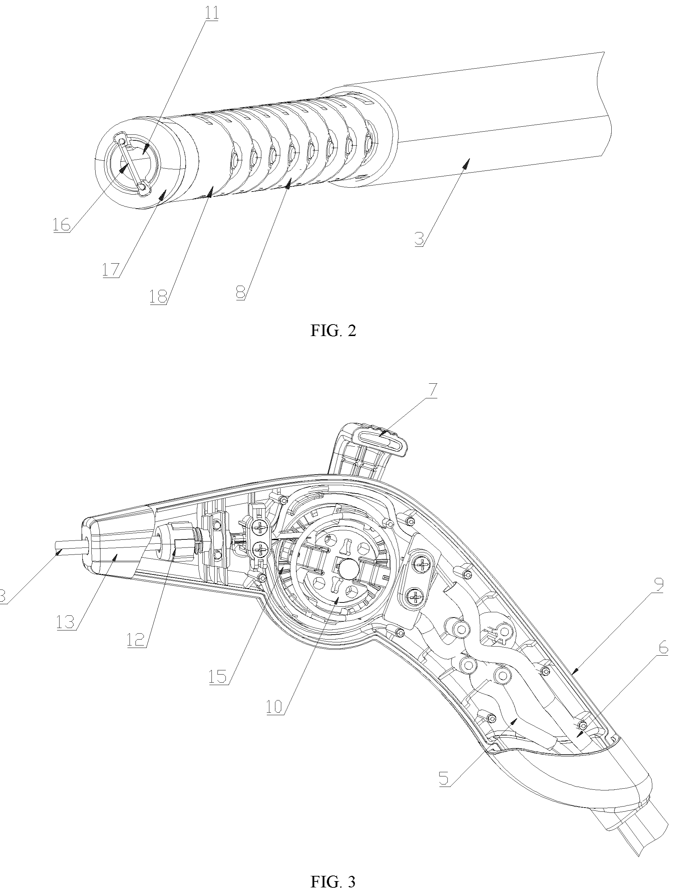

As shown in FIG. 2, specifically, the distal cutter head 4 integrates first working electrode 16, second working electrode 17, and bendable element 8 internally. The bendable element works together with the cutter shank 3 to form a loop electrode. Through this design, the RF plasma system can generate a focused plasma sheath at the first working electrode 16, suitable for high-precision small-area strong cutting. At the second working electrode 17, another discharge loop can achieve large-area tissue cutting and immediate hemostasis, greatly improving surgical efficiency and safety.

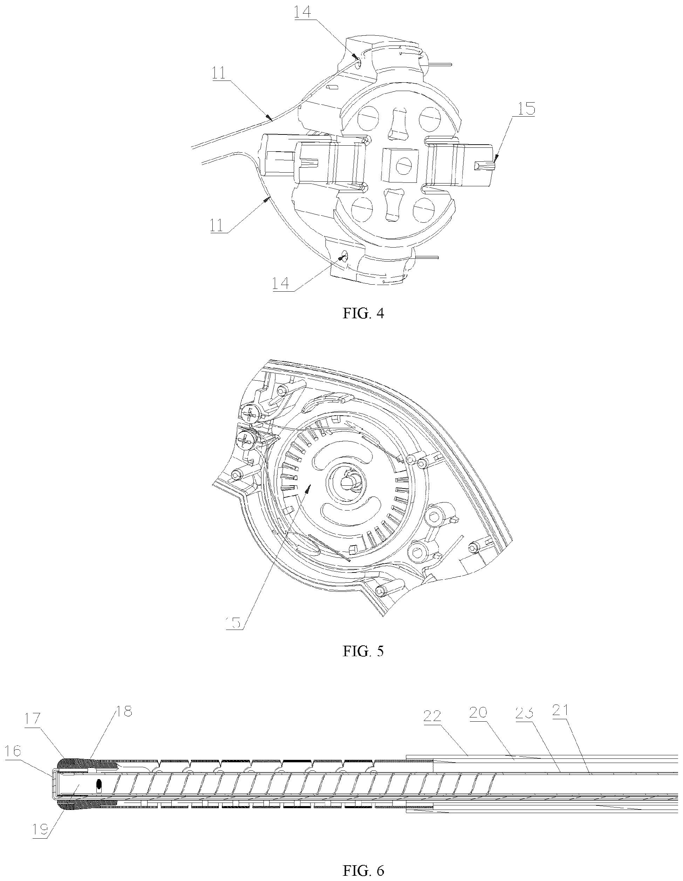

As shown in FIGS. 3 and 4, the proximal handle 2 includes handle housing 9, lever 7, wire reel 10, traction wire 11, cutter shank connector 12, and fastener 13. The wire reel 10 and the lever 7 are fixed with screws. The wire reel 10 is provided with wire hole 14. The traction wire 11 includes one end connected to the bendable device 8 and the other end fixed to the wire hole 14. A tail end of the cutter shank 3 is placed inside the proximal handle 2 and fixed by the cutter shank connector 12 and the fastener 13. Meanwhile, cable 5 and suction tube 6 are separately connected to the cutter shank 3.

As shown in FIG. 3, in order to enhance operational flexibility and precision, the proximal handle 2 is internally provided with an innovative damping adjustment mechanism. This mechanism includes the easy-to-operate lever 7, precisely adjustable damping partition element 15, and the wire reel 10 linked to the damping partition element. The precise control of the bendable element 8 by the traction wire 11 ensures that the distal cutter head 4 can be freely bent and adjusted according to surgical needs, especially suitable for operations in complex cavity environments. The cable assembly 1 is carefully designed to ensure a stable connection with the RF plasma surgical system, and also integrates a negative-pressure suction function. The dual layout of the cable 5 and the suction tube 6 can effectively remove waste during the surgical process and maintain good electrical isolation performance. Especially, the first working electrode 16 cleverly spans an edge of suction hole 19, and the second working electrode 17 is provided around the suction hole, further optimizing the surgical field of view (FOV) and operational efficiency.

As shown in FIG. 6, electrode holder 18 located on an outer edge of the distal cutter head 4 effectively isolates each electrode, ensuring a clear and error-free current path during the surgical process. A double-layer structure of the cutter shank 3, including inner cutter shank 21 and outer cutter shank 20, enhances mechanical strength. In addition, the design of inner insulation layer 23 of the inner cutter shank 21 and outer insulation layer 22 of the outer cutter shank 20 ensures operational safety and stability. In this embodiment, a plurality of damping grooves are formed between partitions of the damping partition element 15. Damping bosses are provided at two ends of the wire reel 10. Through the cooperation between the damping bosses and damping grooves of the damping adjustment mechanism, the bendable degree of the bendable element 8 (as shown in FIGS. 3 and 5) is adjustable, achieving smooth and precise adjustment and improving the operating feel of the surgeon.

Specifically, preferably, the bendable element 8 adopts a serpentine tube as the basic structure to improve the flexibility and durability of the cutter head. The first working electrode 16 (1 to 3 in quantity) is designed as a filamentous structure. The first working electrode 16 and the second working electrode 17 can be made of refractory metals such as molybdenum, tungsten, gold, or platinum and their alloys to further improve equipment performance and extend the lifespan.

During use, in the event of different complex cavity traumas, the lever 7 is pushed to adjust the bending direction of the electrode cutter head 4 in real time. In the a cutting mode, the first working electrode 16 is controlled to form a loop with the loop electrode, and a plasma sheath is concentrated on the first working electrode 16 to perform cutting operations with strong force. In a second coagulate-while-cut mode, the second working electrode 17 is controlled to form a loop with the loop electrode, and the plasma sheath is concentrated on a surface of the second electrode to achieve large-area cutting and hemostasis operations.

In summary, the RF plasma surgical electrode provided in the present disclosure effectively improves the precision and efficiency of surgery through its unique structural design and material selection. Especially, when dealing with complex cavity traumas, the RF plasma surgical electrode provided in the present disclosure has a real-time adjustable bendable characteristic, as well as efficient cutting and hemostasis capabilities.

The above are merely preferred embodiments of the present disclosure, and are not intended to limit the present disclosure. Any modifications, equivalent replacements, improvements, and the like made within the spirit and principle of the present disclosure shall be all included in the protection scope of the present disclosure.

## Claims

1. A radiofrequency (RF) plasma surgical electrode, **characterized by** comprising a cable assembly (1), a proximal handle (2), a cutter shank (3) and a distal cutter head (4) that are connected in sequence, wherein
the distal cutter head (4) is internally provided with a first working electrode (16), a second working electrode (17) and a bendable element (8); the bendable element (8) and the cutter shank (3) form a loop electrode; an RF plasma system, the first working electrode (16) and the loop electrode form a first discharge loop; and the RF plasma system generates a plasma sheath at the first working electrode (16) to achieve a small-area strong cutting operation; and
the RF plasma system, the second working electrode (17) and the loop electrode form a second discharge loop; and the RF plasma system generates a plasma sheath at the second working electrode (17) to achieve a large-area cutting and hemostasis operation.

2. The RF plasma surgical electrode according to claim 1, **characterized in that** the proximal handle (2) is internally provided with an adjustment assembly for adjusting a bending angle of the bendable element (8) in a damping manner; the bendable element (8) is internally provided with a cutting type spring tube communicated with the cutter shank (3); and the cutting type spring tube forms a suction hole (19) at an outer edge of the distal cutter head (4).

3. The RF plasma surgical electrode according to claim 2, **characterized in that** the adjustment assembly comprises a lever (7) provided on a handle housing (9), a damping partition element (15) connected to the lever (7), and a wire reel (10) matched with the damping partition element (15); and a traction wire (11) is provided on the wire reel (10) to pull the bendable element (8) to bend the distal cutter head (4).

4. The RF plasma surgical electrode according to claim 3, **characterized in that** the cable assembly (1) comprises a cable (5) connected to the RF plasma surgical system and a suction tube (6) connected to a negative-pressure suction device; the cable (5) is electrically connected to the loop electrode; the suction tube (6) is separately communicated with the cutter shank (3) and the cutting type spring tube; the first working electrode (16) spans an outer edge of the suction hole (19); and the second working electrode (17) surrounds the suction hole (19).

5. The RF plasma surgical electrode according to claim 1, **characterized in that** an outer edge of the distal cutter head (4) further comprises an electrode holder (18); and the electrode holder (18) separates the first working electrode (16), the second working electrode (17), and the loop electrode.

6. The RF plasma surgical electrode according to claim 2, **characterized in that** the cutter shank (3) comprises an inner cutter shank (21) and an outer cutter shank (20) sleeved onto the inner cutter shank (21); an extension end of the inner cutter shank (21) is connected to the cutting type spring tube; and the outer cutter shank (20) and the bendable element (8) form the loop electrode.

7. The RF plasma surgical electrode according to claim 6, **characterized in that** an end of the proximal handle (2) adjacent to the cutter shank (3) is provided with a cutter shank connector (12) and a fastener (13) for fixing the outer cutter shank (21).

8. The RF plasma surgical electrode according to claim 6, **characterized in that** the inner cutter shank (21) is wrapped in an inner insulation layer (23), and the outer cutter shank (20) is wrapped in an outer insulation layer (22).

9. The RF plasma surgical electrode according to claim 3, **characterized in that** the damping partition element (15) is provided with a plurality of damping grooves arranged at an interval; the wire reel (10) is provided with damping bosses; and different damping bosses are matched with the damping grooves to achieve damping adjustment.
